# EUROPEAN PATENT SPECIFICATION

(11) **EP 3 752 661 B1**
(45) Date of publication and mention of the grant of the patent: **14.10.2026**
(21) Application number: 19703111.5
(22) Date of filing: 11.02.2019
(51) Int. Cl.: C25B 1/46, C25B 15/08, C01D 3/06, C07C 68/02, C07C 68/08, C07C 209/78

(54) **PROCESS FOR THE WORK-UP AND REUSE OF SALT-CONTAINING PROCESS WATER**
VERFAHREN ZUR AUFARBEITUNG UND WIEDERVERWENDUNG VON SALZHALTIGEM PROZESSWASSER
PROCÉDÉ DE TRAITEMENT ET DE VALORISATION D'UNE EAU DE PROCESSUS SALÉE

(30) Priority: 14.02.2018 EP 18156606
(43) Date of publication of application: 23.12.2020
(73) Proprietor: Covestro Deutschland AG, 51373 Leverkusen (DE)
(72) Inventor: SCHIESSER, Yuliya, 53844 Troisdorf (DE); WERNER, Knud, 47800 Krefeld (DE); BULAN, Andreas, 40764 Langenfeld (DE)
(74) Representative: Levpat
(86) International application number: PCT/EP2019/053261
(87) International publication number: WO 2019/158463

(56) References cited:
- EP-A1- 2 669 305
- WO-A1-00/78682
- US-A1- 2010 219 372
- US-B1- 6 340 736

## Description

The invention relates to a process for the work-up of salt-containing process water, in particular from a chemical production process, e.g. process water from the preparation of methylenedi(phenyl isocyanates) (MDI), of polycarbonate by the solution polymerization process (SPC) or of diphenyl carbonate (DPC), with the objective of utilizing the salt obtained from the process water in chloralkali (CA) electrolysis.

**MDI** is an important material in polyurethane chemistry and is usually obtained industrially by phosgenation of the corresponding polyamines of the diphenylmethane series. The preparation of polyamines of the methylenedi(phenylamine) series, hereinafter also referred to as MDA for short, is described in numerous patents and publications. The preparation of MDA is usually carried out by reaction of aniline and formaldehyde in the presence of acid catalysts. Hydrochloric acid is usually used as acid catalyst, and the acid catalyst is, according to the prior art, neutralized and thus consumed by addition of a base, typically aqueous sodium hydroxide, at the end of the process and before the concluding work-up steps, for example the removal of excess aniline by distillation. In general, the neutralizing agent is added in such a way that the neutralization mixture obtained can be separated into an organic phase containing the polyamines of the MDA series and excess aniline and an aqueous phase (MDA process water) which contains sodium chloride together with residues of organic constituents. A procedure of this type is described, for example, in EP 2 096 102 A1.

The preparation of polycarbonate by the solution polymerization process (SPC) is usually carried out by a continuous process, firstly by preparation of phosgene and subsequent reaction of bisphenols and phosgene in the presence of alkali metal hydroxide and a nitrogen catalyst, chain terminators and optionally chain branching agents at the phase interface in a mixture of aqueous-alkaline phase and organic solvent phase.

The preparation of diaryl carbonates (DPC) is usually carried out by a continuous process by preparation of phosgene and subsequent reaction of monophenols and phosgene at the interface in an inert solvent in the presence of alkali metal hydroxide and a basic nitrogen catalyst.

In both processes (SPC, DPC production), the organic, polycarbonate-containing phase is usually separated off from the NaCl-containing reaction water after the reaction, washed with an aqueous liquid (washing water) and separated from the aqueous phase as far as possible after each washing operation. The resulting NaCl-containing reaction water contaminated with residual organics can, separately or in the mixture with washing water, be, for example, stripped by means of steam and can be considered as SPC or DPC process water. This is described by way of example in EP 2 229 343 A1.

The aqueous phases (the process water from MDA, SPC or DPC production) have a sodium chloride content in the range of typically from 5 to 20% by weight (process water) and could in principle be used further in chloralkali electrolysis (CA electrolysis) if they were not contaminated by production-related materials.

It has been found that it is necessary for use of this process water in CA electrolysis to adhere to particular limit values for organic and inorganic impurities in the process water in order to prevent damage to membranes or electrodes by deposits or chemical processes. In order to be able to use the process water reliably in chloralkali electrolysis, hereinafter also referred to as CA electrolysis for short, the proportion of these impurities has to be reduced. It has now been found that, in particular, the concentration of organic impurities (TOC, total organic carbon) in the brine before the electrolyser should if possible not exceed the value of 5 mg/l. Inorganic impurities (Ca, Mg, Si, Mn, Ni, etc.) lead to an increase in the electric potential in the CA electrolysis and should likewise be removed as far as possible.

The process water could thus be mixed, after purification, with anode dilute brine from the CA electrolysis and made up by addition of additional, solid alkali metal chloride salt to the necessary electrolysis entry concentration (e.g. to about 310 g/l of NaCl in the case of NaCl wastewater) and be fed into the electrolysis. However, in the known production processes, large-volume process water streams having a comparatively low NaCl concentration are generally obtained, so that in many cases only part of the total wastewater stream can be recycled in this way since otherwise too much water would be introduced into the CA electrolysis. Simple concentration of the purified process water to the typical electrolysis entry concentration (e.g. about 310 g/l of NaCl) would lead to more process water being able to be recycled, but this at the same time leads to accumulation of the organic and inorganic impurities remaining after purification in the concentrate, so that an additional purification step is necessary in order to achieve the quality for the CA electrolysis.

Processes for treating salt-containing process water have been described in a number of patents.

The patent document CN100506783C discloses a two-stage process for extracting polymethylenepolyphenylpolyamine from salt-containing solutions. The disadvantage of the process lies in the high final concentration of polymethylenepolyphenylpolyamine in the salt solution after the treatment (high TOC and TN values), so that process water treated by this method cannot be used in CA membrane electrolysis.

The reduction of the TOC value of process water from MDI production by oxidation at a pH in the range from 12 to 14 with subsequent treatment with activated carbon is described in CN100534931C and EP2479149B1. The TOC values could be decreased to the range from 6 to 8 mg/l by means of the process. However, a disadvantage of the process lies in the activated carbon treatment. Possible leaching of inorganic ions (Ca, Mg, Si, etc.) from the activated carbon reduces the brine quality again, so that inorganic ions have to be removed again in a complicated manner from the brine.

A possible treatment of MDA process water and utilization in electrolysis is described by EP 2 096 102 A1. The treatment is carried out here by setting to a pH of less than or equal to 8, stripping of the process water with steam, subsequent treatment with activated carbon and concentration or making-up of the solution by means of solid salt (NaCl) to a content of greater than 20% by weight of NaCl. The process does not make it possible to achieve the purity of the process water in respect of organic and inorganic impurities which is, according to present-day knowledge, required for CA electrolysis. In addition, complete recycling of the generally large-volume process water stream will not be possible since a CA membrane electrolysis can take up only a limited amount of additional water.

The treatment of the process water from polycarbonate production has likewise been described a number of times. Here, the objective is to achieve freeing of the process water of organic impurities and concentration to saturation by means of stripping, activated carbon and osmotic distillation (see WO 2017/001513 A1) or catalytic oxidation and evaporation (see US6340736B1). Neither of the treatment processes solve the above-described problem of a large-volume process water stream being able to be recycled completely to a CA membrane electrolysis.

A number of patents describe evaporation and/or crystallization processes for reduction of organic and inorganic impurities in salt-containing solutions. The focus here has been on the removal of inorganic impurities (US20060144715A1), by addition of hydrochloric acid (US9169131B1) or by use of infrared radiation (EP0541114A2). The first publication EP2565159A1 describes a process for freeing industrial salt-containing solutions of organic and inorganic impurities by recrystallization. Owing to the double crystallization step, this process is particularly disadvantageous and energy-consuming because of the double evaporation of water. US2010219372A1 describes a purification of salt solution by means of a combination of various steps, inter alia by optional utilization of a crystallization step. The objective of this combination of various steps is to obtain a salt solution having a TOC content of < 10 ppm and to use this solution in any chemical process. However, this TOC content is far too high for use of the salt solution in chloralkali membrane electrolysis.

US6340736 cites, inter alia, US3655333 in which freeze crystallization is used to purify salt-containing solutions. Here, a contaminated salt solution is saturated (about 26.3% by weight of NaCl) by addition of solid salt, then cooled to from 0 to -20°C, with the saturated salt solution separating into solid NaCl dihydrate (61.9% by weight of NaCl) and a salt solution having a eutectic composition (23.2% by weight of NaCl). The impurities become concentrated in the eutectic solution. The dihydrate is separated off and heated. This forms solid salt and saturated salt solution. A method of decomposing organic compounds in water by treatment with ozone is disclosed in WO 00/78682 A1. The water to be treated comprises preferably 2 to 20 % by weight brine and shall initially have a TOC of more than 2 ppm and shall additionally comprise solubilized carbonic acid or carbonates. After the treatment, the resulting brine can be used as a feedstock for electrolysis to produce chlorine.

It can be seen just from the compositions that not more only than about 8% of dihydrate can be obtained from the saturated salt solution. In the subsequent heating step, just a maximum of about 48% can in turn be obtained as solid salt therefrom, i.e. the total yield is extremely low at a maximum of 3.8%. Such a purification process has therefore not become established industrially.

A further process for treatment of organically polluted salt-containing wastewater is disclosed in CN203295308U. The treatment is carried out essentially by electrochemical oxidation of organic impurities by means of a diamond electrode with subsequent crystallization of the salt. The quality of the treated salt nevertheless does not meet the requirements of CA electrolysis since a purity of 99% by weight is described here as sufficient for the work-up. Proceeding from the prior art indicated above, it is an object of the invention to provide a process in which salt-containing process water is purified and the water content thereof is reduced to such an extent that it can be recycled without problems to a chloralkali electrolysis without the above-described further technical disadvantages, in particular for long-term, trouble-free operation of the electrolysis.

The invention provides a process for the work-up and reuse of salt-containing process water from a production process, in particular from a chemical production process, which contains an alkali metal chloride, preferably sodium chloride, as salt in a concentration of at least 4% by weight and organic or inorganic and organic impurities, wherein
a) the process water is firstly subjected to oxidative and/or adsorptive purification to remove organic impurities,
b) a preconcentrated, purified process water is optionally produced from the purified process water by removal of water to a concentration of not more than 26% by weight of alkali metal chloride in the process water, preferably as desired by means of one or more of the processes: high-pressure reverse osmosis, electrodialysis, evaporation, membrane distillation or vaporization,
c) a partial amount of the purified process water from step a) or b) having a salt concentration of from 4% by weight to 26% by weight, preferably from 7% by weight to 26% by weight, is introduced into the brine circuit of a chloralkali electrolysis,
d) the residual amount of process water remaining from step c) is concentrated further by removal of water
e) and the alkali metal chloride is crystallized out and
f) separated off as solid alkali metal chloride from the mother liquor and purified by means of a wash, so that the solid alkali metal chloride, analysed after dissolution in deionized pure water in a concentration of 300 g/l, has a TOC content of not more than 1 mg/l,
g) the solid, purified alkali metal chloride from step f) is introduced into the brine stream of the chloralkali electrolysis,
h) the products obtained from the alkali metal chloride electrolysis after step g) and optionally c): chlorine, alkali metal hydroxide, preferably sodium hydroxide, and optionally hydrogen are recirculated as desired again to the production process.

Recirculation as desired of the products chlorine, alkali metal hydroxide, preferably sodium hydroxide, and optionally hydrogen here means that each of these products can be reused independently in the initial chemical production process. As an alternative, the respective product is utilized in another way.

The novel process is preferably applied to process water in which the concentration of salt, in particular of alkali metal chloride, in the process water before step a) is at least 6% by weight, preferably at least 8% by weight, particularly preferably at least 12% by weight.

In the novel process, the alkali metal chloride is preferably sodium chloride and the alkali metal hydroxide is preferably sodium hydroxide.

The deionized water used in step f) has, in particular, a TOC of not more than 0.01 mg/l.

A production process which is particularly suitable for carrying out the novel process and from which the process water is taken is a process for the preparation of polycarbonates or of polycarbonate precursors, in particular diphenyl carbonate, or of isocyanates, in particular methylene di(phenylisocyanates) (MDI), or of methylenedi(phenylamine) (MDA).

The organic impurities with which the process water worked up using the novel process is contaminated are, in particular, compounds selected from the group consisting of: aniline, MDA and precursors thereof, formaldehyde, methanol, phenol or others, as described below.

The inorganic impurities with which the process water which is worked up using the novel process is contaminated are, in particular, compounds selected from the group consisting of: salts of the cations of the metals: Ca, Mg, Fe, Al, Si, B, Sc, Ba, Ti, Cr, Mn, Ni and Ru in combination with anions, in particular anions selected from the group consisting of: Cl⁻ , Br⁻, F⁻, SiO₄²⁻, SO₄²⁻.

The optional oxidative purification in step a) to remove organic impurities is preferably carried out by treatment with ozone at an initial pH of the process water which is to be set to at least 1 and a temperature of at least 35°C, preferably at least 50°C.

In a particularly preferred embodiment of the novel process, the amount of ozone is not more than 2 g of ozone per litre of process water.

A further preferred variant of the novel process is characterized in that the adsorptive purification in step a) to remove organic impurities is carried out by means of adsorption on activated carbon, adsorber resins or zeolites.

In a preferred variant of the novel process, the oxidative purification, i.e. removal of organic impurities from the process water, in step a) is optionally carried out in addition to another oxidative purification or solely by means of electrochemical anodic reaction at a diamond electrode, preferably at a boron-doped diamond electrode.

The removal of organic impurities in the prepurification after step a) is particularly preferably carried out down to a residual content of impurities of not more than 5 mg/l of TOC.

A further preferred embodiment of the novel process is characterized in that the optional preconcentration after step b) is carried out to a concentration of not more than 26% by weight of alkali metal chloride in the process water.

Another preferred variant of the novel process is characterized in that the water obtained in the preconcentration in the optional step b) is reused for dilution of alkali metal hydroxide solution, preferably of sodium hydroxide solution, for the chemical production process from which the process water has been taken, in particular for a process for the preparation of polycarbonates, polycarbonate precursor or MDA.

The water obtained in the concentration and crystallization in steps d) and e) can, in another preferred variant of the novel process, be used further for diluting alkali metal hydroxide solution, preferably sodium hydroxide solution, for the chemical production process, in particular a process for the preparation of polycarbonates, polycarbonate precursor or MDA.

The solid alkali metal chloride obtained in the crystallization in step f) is, in a particularly preferred embodiment of the novel process, washed by means of deionized water and/or purified alkali metal chloride solution (TOC content preferably not more than 5 mg/l), preferably in countercurrent, to effect purification before further use.

In a further particularly preferred embodiment of the novel process, a purified alkali metal chloride solution from a substream of the process water purified in step a) and/or water which is removed and obtained in the optional preconcentration according to step b) and/or the concentration according to step d) or e) is used for the washing of the solid salt in step f).

In another preferred embodiment, an alkali metal chloride solution for which purified alkali metal chloride salt is dissolved in water which is removed and obtained in the performance of step b) and/or d) is used for washing of the salt in step f). This has the advantage that particularly pure alkali metal chloride solution (e.g. having a TOC content of < 2 mg/l) is used for the wash.

In a further preferred embodiment of the novel process, the mother liquor which has been separated off from the alkali metal chloride in step f) is divided into two streams, and the one larger stream is recirculated to the concentration operation in step d) and the other, smaller substream amounting to not more than 5% by weight of the mother liquor which has been separated off is disposed of. This is necessary particularly in the continuous mode of operation since otherwise the circulated mother liquor always continues to accumulate impurities.

Particular preference is also given to a process which is characterized in that, based on 100 parts by weight of alkali metal chloride separated off, from 5 to 20 parts by weight of washing liquid are used for the optional washing of the solid alkali metal chloride in step f).

Process water obtained in the production of MDA, which can be treated by means of the novel process, should be freed of organic impurities still present before use in chloralkali membrane electrolysis. Typical possible impurities are, in particular, aniline, MDA and precursor compounds thereof, formaldehyde, methanol and traces of phenol, with methanol being able to get into the process as contaminant of the formaldehyde and phenol being able to get into the process as contaminant of the aniline. Further typical impurities are formate, alcohols, amines, carboxylic acids and alkanes. The total concentration of the organic impurities varies, depending on the method of preparation, from, in particular, 50 to 100 mg/l of TOC. The MDA process water usually has, as a function of the production method, a pH in the range from 12 to 14 and has a typical concentration of sodium chloride in the range from 10% by weight to 15% by weight. The temperature can be in the range from 40 to 60°C.

Possible main impurities in the process water from polycarbonate production, which can be treated by means of the novel process, are typically phenol, bisphenol A, phenol derivatives and benzene derivatives having different alkyl substitutions and also halogenated aromatics, preferably from the group consisting of butylphenols, isopropylphenols, trichlorophenols, bromophenols and also aliphatic amines and salts thereof (trimethylamines, butylamines, dimethylbenzylamines) and also ammonium compounds and ammonium salts thereof, preferably trimethylamines, butylamines, dimethylbenzylamines, ethylpiperidine and quaternary ammonium salts thereof. The process water from diphenyl carbonate (DPC) and polycarbonate production by the phase interface process (referred to as SPC production for short) usually has, as a function of the method of production, a pH in the range from 12 to 14 and has a typical concentration of sodium chloride in the range from 5 to 7% by weight (for SPC processes) and from 14 to 17% by weight (for DPC processes) and a temperature of about 30°C.

Phenol and derivatives thereof, bisphenol A and further high molecular weight organic compounds are chlorinated in chloralkali electrolysis and form AOX (adsorbable organic halogen compounds). Ammonium compounds and salts thereof and also all amines lead to formation of NCl₃ and also a voltage increase in the chloralkali electrolysis voltage. Aniline and MDA are readily oxidizable in chloralkali electrolysis and lead immediately to formation of aniline black, which blocks membranes and electrodes. Formate leads to contamination of the chlorine with CO₂.

A particularly preferred variant of the novel process is therefore characterized in that the organic impurities are compounds selected from the group consisting of: aniline, MDA and precursors thereof, formaldehyde, methanol, phenol or bisphenol A, phenol derivatives and benzene derivatives having different alkyl substitutions and also halogenated aromatics (for example butylphenol, isopropylphenol, trichlorophenol, dibromophenol) and also polar, aliphatic amines and salts thereof (trimethylamines, butylamines, dimethylbenzylamines) and also ammonium compounds and salts thereof.

The objective of the prepurification according to step a) in the novel process is recycling of salt-containing process water in order to very largely avoid disposal of the process water in its entirety; this applies both in respect of the alkali metal chloride salt with the possibility of utilization thereof in the electrolysis for the production of chlorine and also in respect of the water for reuse thereof in chemical production. The process water comprises organic and inorganic impurities as described in detail above, which should be removed. Accumulation of the impurities in a recirculation process would otherwise lead to a reduction in the product quality of the production processes and to possible damage to the production plants. Ideally, both the salt present in the process water and also the water should acquire the quality necessary for reuse during the recycling process. The removal of impurities can be effected in various ways and at various points in the process. Ideally, the usually unavoidable amount of process water to be disposed of should be minimized as far as possible. Both water and salt in the process water are valuable materials for reuse. The disposal of process water is therefore not economical.

MDA and aniline (constituents of the MDA process water) have been identified as particularly damaging substances for CA electrolysis. In order to prevent accumulation of these substances, for example in CA membrane electrolysis, these should be removed or destroyed in a demonstrable manner. Oxidation with the aim of mineralizing as much as possible of the substances to CO₂ and water has been found to be the best-suited method. Firstly, it ensures that no aniline and MDA get into the CA electrolysis. Secondly, further organic impurities present in the MDA process water are also mineralized to CO₂ and water, so that the total amount of TOC and therefore also the amount to be disposed of can be minimized.

The purification of the alkali metal chloride-containing solution obtained in the MDA processes employed can be carried out separately (reaction water) or, as set forth in DE10 2008 012 037 A1, together with other water streams (washing water). Preference is given to the water streams obtained in MDA production being combined and purified together.

Ozonolysis is a widespread method for sterilization and disinfection of drinking water. The method is also being increasingly used in wastewater purification for oxidation of problematical microimpurities such as pharmaceuticals, crop protection agents or cosmetics, with the objective here being to oxidize the organic impurities only to such an extent that they can subsequently be passed to biological purification.

The mechanisms of action of ozone in the degradation of organic substances at various pH values are known. In the acidic and neutral range, the ozone molecule is added predominantly onto double bonds, and after the subsequent hydrolysis the molecule is broken up. The reaction occurs selectively with different degradation rates. Organic acids and ketones are usually formed here. In alkaline medium, very rapid oxidation is brought about by free OH radical formation. The free OH radicals formed have a free outer electron which serves as strong oxidizing agent. The reaction occurs unspecifically in this case. Oxidation in alkaline medium is therefore recommended in the literature for mineralizing organic impurities to CO₂ and water. The degradation of aniline is, for example, increased from 58% at pH 3 to 97% at pH 11, while COD (chemical oxygen demand, overall parameter as measure of the sum of all materials which are present in the water and are oxidizable under particular conditions) is removed to an extent of from 31% to 80% (Journal of Chemistry, Volume 2015, Article ID 905921, 6 pages, http://dx.doi.org/10.1155/2015/905921, Degradation Characteristics of Aniline with Ozonation and Subsequent Treatment Analysis). Phenol could, for example, be degraded to an extent of 100% at pH 9.4, but only to an extent of 85% at pH 3 (S. Esplugas et al. / Water Research 36 (2002) 1034-1042, Comparison of different advanced oxidation processes for phenol degradation).

Ozone oxidizes pollutants (e.g. AOX, adsorbable organically bound halogens). In the case of water having a high salt content, it is possible for, for example, chlorine ions to be oxidized to chlorine and for these to react with organic compounds and thus reform AOX.

The water solubility and the half life of ozone are known to be very greatly temperature-dependent. Thus, the solubility of ozone in water at 45°C is virtually zero. On the other hand, the reaction rate increases by a factor from two to three at a temperature increase of 10°C. In general, a maximum possible temperature in an ozonized water system of about 40°C is recommended; above this, degradation of ozone occurs too rapidly.

It has surprisingly been found, in particular, that the oxidation has led to virtually complete mineralization of all organic impurities at a pH of less than or equal to 8 and a relatively high temperature (50-75°C) (example at various pH values and various T). In a preferred process, the pH is lowered by means of hydrochloric acid or hydrogen chloride.

The prepurification of DPC and SPC process water can be carried out, in particular, by treatment with activated carbon at a pH of equal to or less than 8, as is known in principle from the prior art (see, for example, EP 2 229 343 A1). As an alternative, other adsorbents (zeolites, macroporous and mesoporous synthetic resins, zeolites etc.) can be used.

Crystallization as additional purification step is an important process in the overall process. The following aspects should preferably be taken into account in the crystallization and in carrying out the novel process:
1) the quality of the purified salt should preferably attain the TOC value of less than 5 mg/l necessary for the electrolysis;
2) the residual TOC content should particularly preferably not comprise any substances which are damaging to the electrolysis (these could also accumulate in the electrolysis circuit);
3) the water separated off in the novel process (steps b) or d)) should preferably as far as possible not comprise any residual impurities (TOC preferably below 2 mg/l) (e.g. because of the risk of deposition of TOC components in the compressor which is used in evaporation with mechanical vapour compression or because of the risk of contamination of the salt during the wash in step f)).

It has been found, in particular, that prepurification of the process water from various polymer production processes (here, for example, MDA and DPC production) is necessary for a number of reasons. Firstly, it has been found that yellow discolouration of the salt owing to the oxidation of MDA and formation of aniline black has occurred during the crystallization process of concentrated MDA process water despite the attained specification value of 3.5 mg/l of TOC in the crystallized material (Example 1). Furthermore, the distillate phase from DPC/SPC/MDA process water has a high proportion of organic impurities (see Examples 1 and 2).

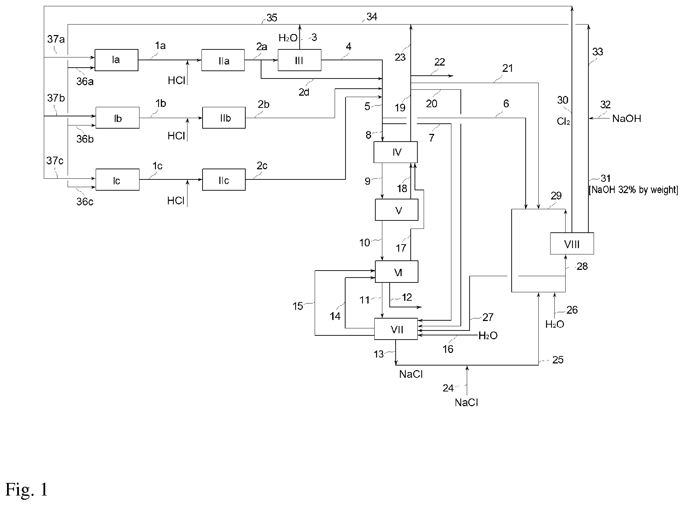

The invention will be illustrated in more detail by way of example below with the aid of Figure 1 without being restricted thereto.

Figure 1 schematically shows the process of the invention with concentration of process water from different sources (MDA, SPC and DPC production) by evaporation and crystallization.

### List of reference numerals:

- Ia: SPC production
- Ib: DPC production
- Ic: MDA production
- IIa: adsorptive prepurification
- IIb: adsorptive prepurification
- IIc: oxidative prepurification
- III: preconcentration
- IV: heat exchanger
- V: evaporation stage
- VI: crystallization
- VII: liquid/solid separator and salt wash
- VIII: chloralkali membrane electrolysis
- 1a: SPC process water
- 1b: DPC process water
- 1c: MDA process water
- 2a, 2d: prepurified SPC process water
- 2b: prepurified DPC process water
- 2c: prepurified MDA process water
- 3: deionized process water from preconcentration stage III
- 4: preconcentrated process water from preconcentration stage III
- 5: mixed process water from 2a, 2b, 2c, 2d and 4
- 6: prepurified process water (substream)
- 7: prepurified process water (substream)
- 8: feed stream
- 9: preheated salt solution
- 10: evaporated salt solution
- 11: concentrated salt solution with salt
- 12: mother liquor (purge)
- 13: salt
- 14: mother liquor
- 15: loaded washing water
- 16: deionized water
- 17: distillate from crystallization VI
- 18: distillate from evaporation V
- 19: distillate (total stream from heat exchanger IV)
- 20: distillate (substream) used as washing water
- 21: distillate (substream)
- 22: distillate (residual stream)
- 23: distillate (substream)
- 24: solid additional salt
- 25: concentrated salt stream
- 26: water
- 27: salt solution
- 28: finished salt solution to electrolysis
- 29: dilute brine
- 30: chlorine
- 31: sodium hydroxide
- 32: sodium hydroxide (addition)
- 33: sodium hydroxide
- 34: diluted sodium hydroxide
- 35: diluted sodium hydroxide
- 36 a, b, c: sodium hydroxide entry streams for SPC, DPC and MDA production
- 37 a, b, c: chlorine entry streams for SPC, DPC and MDA production

### Examples:

### General description of the work-up and concentration of process water from various sources

The work-up and concentration of process water can be carried out by evaporation and crystallization of the various prepurified process waters either separately or together according to the scheme as depicted in Fig. 1.

Figure 1 schematically shows the process of the invention with concentration of process water from different sources (MDA, SPC and DPC production) by evaporation and crystallization.

In the case of SPC production (Ia), the process water 1a is formed and is firstly brought to a pH of less than 8 using hydrochloric acid (HCl) and then prepurified by means of activated carbon (IIa). The prepurified stream 2a can optionally be preconcentrated (III) to form a stream 4 and then be introduced in the mixed process water 5 or can be introduced directly (2d) into the mixed process water 5.

In the case of DPC production (Ib), the process water 1b is formed and is likewise brought to a pH of less than 8 using hydrochloric acid (HCl), then prepurified by means of activated carbon (IIb) and introduced as stream 2b into the mixed process water 5.

In the case of MDA production (Ic), the process water 1c is formed and is also brought to a suitable pH value using hydrochloric acid (HCl), then oxidatively prepurified (IIc) and introduced as stream 2c into the mixed process water 5.

A substream 6 is taken from the mixed process water 5 and fed into the brine circuit of the electrolysis VIII. A further substream 7 can optionally be fed to the solid/liquid separator VII for salt washing. The remaining mixed process water is then introduced as feed stream 8 into the heat exchanger IV and be preheated therein.

The hot distillate 18 or 17 from the evaporation stage V (stream 18) or the crystallization VI (stream 17) is preferably used for this purpose. In the subsequent evaporation stage V and the crystallization stage VI, water is withdrawn as distillate 17 or 18 by evaporation to form the brine streams 9 and 10. The amount of water evaporated depends on the concentration of impurities in the feed stream 8. In general, more than 95% of the water can be withdrawn from the feed stream 8. Depending on the size of the feed stream 8, it can also be useful to carry out evaporation and crystallization in a single apparatus (not shown).

The evaporated water is compressed by means of compressors and used for heating the evaporation (stage V) or the crystallization (stage VI) (mechanical vapour compression; not shown here). As an alternative, however, in the case of a multistage process procedure, it can be fed directly into the next stage of a multistage evaporation or crystallization plant to effect heating (not shown here). The condensate 17 or 18 (distillate) formed from the steam is used for preheating the feed stream 8 in the heat exchanger IV. Since the TOC content of the distillate 17 or 18 is below 5 mg/l due to the prepurification of the feed stream 8, it can, after feed preheating, be used in a process requiring a particular purity, e.g. a chloralkali membrane electrolysis VIII (stream 21).

The evaporation and crystallization VI forms a mixture 11 of solid salt and mother liquor saturated with NaCl. The mother liquor comprises a major part of the organic and inorganic impurities. For this reason, part of the mother liquor remaining after the crystallization (stream 12, purge) is discharged together with the major part of the impurities present therein from the crystallization stage VI and discarded.

Part of the mother liquor 14 is separated off from the mixture 11 in the separator VII and recirculated to the crystallization step VI.

The solid salt with residual adhering mother liquor is washed with distillate (substream 20) as washing water in stage VII and obtained as clean salt 13. It is particularly advantageous to carry out a countercurrent wash with the distillate (stream 20) in stage VII: the particularly pure stream 20 is used for the second washing step for the solid salt. In this second washing step in stage VII, the stream 20 takes up residual impurities from the surface of the solid salt. The loaded washing water is collected and used for the first washing step in stage VII. Here, it displaces the residual adhering mother liquor and takes up additional further impurities. Since the washing water also becomes loaded with salt, it is likewise recirculated as loaded washing water 15 after the wash in stage VII to the crystallization VI.

As an alternative, washing of the salt in stage VII or the countercurrent wash can also be carried out using fresh water, preferably demineralized or deionized water (stream 16) instead of the distillate 20.

Since the water becomes loaded with salt in the wash or countercurrent wash in stage VII, prepurified process water (stream 7) can optionally also be used, as shown in Figure 1. Since this already contains salt, the loss of crystallized salt in the wash is lower.

The use of the salt solution having the electrolysis entry concentration (stream 27) is particularly advantageous since virtually no crystallized salt dissolves in this case.

Based on 100 parts by weight of alkali metal chloride which has been separated off, the amount of the washing liquid is 10 parts by weight.

As a result of the crystallization and the countercurrent wash, the salt obtained is provided in a purity required for the CA electrolysis VIII. The TOC value is preferably less than or equal to 5 mg/l in the saturated solution.

Since, inter alia, part of the salt is discharged together with the mother liquor (stream 12) and discarded, a partial amount of salt has to be added as supplement (stream 24) in order to provide a sufficient amount of chlorine for the processes Ia - Ic from the CA electrolysis (step VIII). The amount of salt originating from the evaporation/crystallization step (stream 13) and this supplement 24 are fed as stream 25 to the CA electrolysis VIII. Depending on the requirements of the electrolysis VIII, it can be necessary to introduce fresh water (stream 26) in order to produce the starting solution 28 for the electrolysis VIII.

In order to cover the water requirement of the CA electrolysis, part 6 of the prepurified process water can, as an alternative, be fed directly into the electrolysis VIII. The salt requirement is covered by the crystallized salt 13 and the salt 24 introduced from the outside. The streams are mixed with the dilute brine 29 so as to give a brine concentration of about 300 g/l of NaCl. The TOC content of the mixture must not exceed 5 mg/l.

The chlorine 30 formed in the electrolysis is used for the production processes SPC, DPC and MDI (chlorine entry streams 37a, 37b, 37c). The sodium hydroxide 31 formed is likewise used there. The requirement going beyond the sodium hydroxide formed is if necessary provided by introduction of external sodium hydroxide 32.

Since the total sodium hydroxide stream 33 is usually used as dilute feed streams 36a, 36b, 36c in the production processes Ia, Ib and Ic, a substream 23 of the distillate 19 and the permeate 3 from the preconcentration can be used for producing dilute alkali (streams 34, 35). The excess water 22 can be used for other purposes in production processes.

### Example 1 (comparative example)

### Crystallization without prepurification, salt water from MDA production:

A polluted sodium chloride solution (starting solution) which simulates a typical MDA process water and has the following composition: 135 g/l of NaCl, 132 mg/l of formate, 0.56 mg/l of aniline, 11.6 mg/l of MDA, 30 mg/l of phenol was used. About 1.155 l of water (distillate) were withdrawn from 1.5 litres of this solution at a vaporization rate of 12 ml/min while stirring continually. This corresponds to about 80% of the proportion of water in the starting solution. The remaining concentrate containing the solid was separated on a suction filter into the mother liquor and solid salt (wet). The solid salt separated off was subsequently washed with high-purity brine (pure washing brine) in the suction filter and collected (washing brine). The washed salt was dried at about 100°C in a drying oven. For analytical purposes, 30 g of the dried salt were subsequently taken up in deionized pure water until a solution having the NaCl concentration of 300 g/l (brine) was formed. The measured values from the analyses carried out are summarized in Table 1. As can be seen from Table 1, although the TOC value was below the value of 5 mg/l required for the chloralkali electrolysis as a result of the crystallization and washing procedure, a yellowish discoloration of the salt obtained after the drying procedure was observed. The yellowish discoloration is attributable to the oxidation of the MDA. For use of the salt obtained in this way, this would mean that the CA electrolysis would be damaged over time by MDA oxidation products. Furthermore, part of the organic impurities goes into the distillate (TOC 13 mg/l), which would prohibit direct reuse of the distillate in production processes.

**Table 1**

| **MDA without prepurification** | Amount | NaCl [g/l] | TOC [mg/l] | pH |
|---|---|---|---|---|
| Starting solution | 1.51 | 135 | 52 | 12 |
| Distillate | 1.1551 | 0 | 13 | 6.1 |
| Mother liquor | 265 ml | 310 | 131 | 10.4 |
| Solid salt (wet) | 121.5 g | - | - | - |
| Pure washing brine | 48 ml | 310 | < 1 | - |
| Washing brine | 48 ml | 310 | 91.5 | - |
| Brine for analysis | 100 ml | 300 | 3.5 | 8 |

### Example 2

### Example for sole crystallization and wash without prepurification with countercurrent washing of the salt produced, salt water from DPC production (comparison):

3 litres of DPC process water after neutralization (pH 7.3) were used as initial charge (starting solution). About 2.679 l of water (distillate) were withdrawn from the initial charge (DPC process water) at an evaporation rate of 12 ml/min while stirring continually. This corresponds to about 95% of the proportion of water in the initial process water. The remaining concentrate was separated on a suction filter into the mother liquor and solid salt (wet). A two-stage countercurrent wash was then carried out using washing brine.

In the countercurrent wash, pure washing brine is brought into contact with the salt which has already been washed once, so that the salt is washed a second time. The filtrate then obtained from the pure washing brine is reused for the first washing of the salt.

This in-principle procedure can be approximated as follows:
The solid salt which had been separated off was divided into two approximately equal partial amounts (salt S1 and salt S2). Pure washing brine was likewise divided into two equal parts (pure washing brine RW1 and pure washing brine RW2). The salt S1 was subsequently washed with pure washing brine RW1 on the suction filter. The filtrate was collected as washing brine WS1.1. Washing brine WS1.1 thus represents an approximation of the filtrate which is reused for the first washing of the salt. For this reason, the salt S2 was subsequently washed with the washing brine WS1.1, resulting in the washing brine WS1.2 as filtrate. Finally, the pure washing brine RW2 was used for renewed washing of the salt S2, forming a washing brine WS2. The salt S1 which had been washed once and the twice-washed salt S2 were dried at about 100°C in a drying oven. For analytical purposes, 30 g of the dried salt S1 and 30 g of the dried salt S2 were in each case subsequently taken up in deionized water to give 100 ml of solution, so that a brine containing 300 g of NaCl/L was formed. The measured values for the various fractions are summarized in Table 2. The quality of the brines Br1 and Br2 produced was found to be comparatively good. Nevertheless, a large amount of TOC was found in the distillate (about 80% of the TOC burden) in the experiment. This experiment showed that sole crystallization and washing of the salt formed is not sufficient to provide distillate having a quality sufficient to allow reuse, as in Fig. 1.

**Table 2**

| **DPC without prepurification** | Amount | NaCl [g/l] | TOC [mg/l] | pH |
|---|---|---|---|---|
| Starting solution | 31 | 177 | 25 | 7.3 |
| Distillate | 2.6791 | 1.5 | 22.5 | 6.8 |
| Mother liquor | 124 ml | 300 | 99.8 | 9.7 |
| Solid salt (wet) | 535 g | - | - | - |
| Salt S1 (wet) | 272 g | - | - | - |
| Salt S2 (wet) | 263 g | - | - | - |
| Pure washing brine RW1 | 60 ml | 310 | < 1 | - |
| Pure washing brine RW2 | 60 | 310 | < 1 | - |
| Washing brine WS1.1 | 64 ml | - | - | - |
| Washing brine WS1.2 | 70 | 310 | 51 | 9.6 |
| Washing brine WS2 | 58 | 310 | 27 | 9.6 |
| Brine Br1 | 100 ml | 296 | 1.5 | 9.5 |
| Brine Br2 | 100 ml | 294 | < 1 | 9.4 |

The examples presented show that prepurification of the process water is necessary. Owing to the different chemical natures of the impurities, different purification methods have to be employed to remove organics from the process waters.

### Example 3 (process for prepurification as per step a) (stage IIc) according to the invention)

### Prepurification of MDA process water using ozone at various pH values:

Mixed MDA process water 1c (reaction and washing water) having a TOC content in the order of 70 mg/l (for values, see Table 3) was subjected to O₃ oxidation IIc at various pH values. Firstly, the ozonolysis of original MDA process water having an initial pH of pH 13.1 was carried out. Two further samples were brought by means of HCl to a pH of 7 and 3.4 and ozonized. For the ozonolysis, 3.5 l in each case of process water were firstly brought to a temperature of 75°C in a double-walled glass reactor with continual stirring. An ozone generator COM-AD-01 from Anseros was used for generation of the ozone. The ozone generator setting was kept constant in all tests: oxygen volume flow at inlet 100 l/h; generator power 80% (corresponds to about 3.5 g of ozone per hour). The ozone/oxygen mixture was fed into the glass reactor and mixed with process water. To monitor the experiment, samples were taken every 15 minutes and both TOC and pH were measured. The important parameters and results are summarized in Table 3.

**Table 3:**

| **Time [min]** | **g of O₃/l** | **Initial pH 3** | | **Initial pH 7** | | **Initial pH 13** | |
|---|---|---|---|---|---|---|---|
| | | **pH** | **TOC [mg/l]** | **pH** | **TOC [mg/l]** | **pH** | **TOC [mg/l]** |
| 0 | 0 | 3.4 | 73.5 | 7 | 68.4 | 13.1 | 69.7 |
| 15 | 0.25 | 3.5 | 62.9 | 7.8 | 35.4 | 13.1 | 37.9 |
| 30 | 0.50 | 3.9 | 43.8 | 8 | 18.8 | 13.1 | 21.3 |
| 45 | 0.75 | 6.9 | 23.7 | 8.3 | 10.1 | 13.1 | 14.4 |
| 60 | 1.00 | 7.5 | 14.6 | 8.4 | 7.3 | 13.1 | 11.5 |
| 75 | 1.25 | 7.9 | 8.2 | 8.5 | 5.7 | 13.1 | 10.6 |
| 90 | 1.50 | 8.1 | 5.7 | 8.5 | 5 | 13.1 | 10.4 |
| 105 | 1.75 | 8.3 | 3.2 | 8.6 | 2.8 | 13.1 | 10.1 |
| 120 | 2.00 | 8.3 | 1.9 | 8.7 | 3.15 | 13.1 | 10.5 |

### Example 4 (process for prepurification; stage IIc according to the invention)

### Prepurification of MDA process water using O₃ at various temperatures:

Mixed MDA process water (reaction and washing water) Ic was subjected to an O₃ oxidation IIc at various temperatures (25°C, 50°C and 75°C). Ozonolysis was carried out at an initial pH of 7.7. The important parameters and results are summarized in Table 4. As can be seen from Table 4, the TOC degradation rate at 75°C is better than that at 25°C by a factor of about 2.

**Table 4**

| **Time [min]** | **g of O₃/l** | **25°C** | | **50°C** | | **75°C** | |
|---|---|---|---|---|---|---|---|
| | | **pH** | **TOC [mg/l]** | **pH** | **TOC [mg/l]** | **pH** | **TOC [mg/l]** |
| 0 | 0 | 7.7 | 72 | 7.7 | 72 | 7.7 | 72 |
| 15 | 0.25 | 7.9 | 56 | 8.2 | 45 | 8.4 | 48 |
| 30 | 0.50 | 7.9 | 43 | 8.3 | 42 | 8.4 | 28 |
| 45 | 0.75 | 8.0 | 37 | 8.3 | 28 | 8.5 | 21 |
| 60 | 1.00 | 8.0 | 33 | 8.3 | 24 | 8.5 | 17 |
| 75 | 1.25 | 8.0 | 27 | 8.2 | 22 | 8.5 | 13 |
| 90 | 1.50 | 8.0 | 25 | 8.2 | 16 | 8.5 | 12 |
| 105 | 1.75 | 8.0 | 22 | 8.2 | 15 | 8.6 | 11 |
| 120 | 2.00 | 8.1 | 20 | 8.3 | 11 | 8.6 | 9 |

### Example 5 (stage VI according to the invention)

**Crystallization after the prepurification of MDA process water:** MDA process water 2c (starting solution) after prepurification by means of the ozonolysis IIc (pH after ozonolysis 8.1) was used. 3 litres of MDA process water 2c were treated in a manner analogous to the procedure in **Example 2 (crystallization without prepurification DPC).** The measured values for the process materials are summarized in Table 5 below. In the experiment, a small amount of TOC was found in the distillate (about 7.9% TOC burden). The quality of the brines Br1 and Br2 produced was found to be excellent.

**Table 5**

| **MDA after prepurification** | Amount | NaCl [g/l] | TOC [mg/l] | pH |
|---|---|---|---|---|
| Starting solution | 31 | 153 | 11.3 | 8.1 |
| Distillate | 2.6931 | 1 | 1 | 4.9 |
| Mother liquor | 147 ml | 310 | 161.8 | 9.6 |
| Solid salt (wet) | 433 g | - | - | - |
| Salt S1 (wet) | 216 g | - | - | - |
| Salt S2 (wet) | 217 g | - | - | - |
| Pure washing brine RW1 | 60 ml | 310 | < 1 | - |
| Pure washing brine RW2 | 60 ml | 310 | < 1 | - |
| Washing brine WS1.1 | 51 ml | 310 | - | - |
| Washing brine WS1.2 | 46 ml | 310 | 72.7 | - |
| Washing brine WS2 | 59 ml | 310 | 56.5 | - |
| Brine Br1: 30 g of salt 1 + deionized water to 100 ml | 100 ml | 296 | < 1 | 8.8 |
| Brine Br2: 30 g of salt 2 + deionized water to 100 ml | 100 ml | 296 | < 1 | 8.8 |

In addition, it was surprisingly found in the experiment that inorganic ions also mostly remain in the mother liquor or can be removed by salt washing (Table 6). Here, the masses of the ions in the 3 litres of the starting solution used were determined from the ion concentrations measured in the starting solution and entered in the table. The masses of the ions which would be present in salt S1 and salt S2 after corresponding double washing were calculated from the measured ion concentrations in brine Br2. For this purpose, the volume of the brine Br2 was converted according to the amounts of salt S1 + salt S2: 100 ml / 30 g * (216 g + 217 g) = 1.443 ml.

**Table 6**

| **Ion** | **Sr** | **Ni** | **Ru** |
|---|---|---|---|
| **Unit** | **mg** | **mg** | **mg** |
| 3 1 of starting solution (153 g/l of NaCl) | 0.172 | < 0.03 | < 0.073 |
| Brine 2 (296 g/l of NaCl), converted to all of salt 1 and salt 2: 100 ml/30 g * (216 g+217 g) = 1.443 ml | 0.075 | < 0.005 | < 0.016 |

### Example 7 (stage VI according to the invention)

### Crystallization of salt water from DPC production after prepurification:

DPC process water Ib (starting solution) after the prepurification IIb by means of activated carbon (pH 7.5) was used. 3 litres of DPC process water 2b were treated in a manner analogous to the procedure in **Example 2** (crystallization without prepurification of DPC). The measured values for the process materials are summarized in Table 7. No TOC (measurement limit less than 0.5 mg/l) was found in the distillate in the experiment. The quality of the brines Br1 and Br2 produced was found to be excellent.

**Table 7**

| **DPC after prepurification** | Amount | NaCl [g/l] | TOC [mg/l] | pH |
|---|---|---|---|---|
| Starting solution | 31 | 169 | ca. 1 | 7.5 |
| Distillate | 2.6621 | 1.5 | not detectable | 5.2 |
| Mother liquor | 164 ml | 310 | 12.1 | 8.9 |
| Solid salt (wet) | 473 g | - | - | - |
| Pure washing brine RW1 | 60 ml | 310 | < 1 | - |
| Pure washing brine RW2 | 60 ml | 310 | < 1 | - |
| Washing brine WS1.1 | 55 ml | 310 | - | - |
| Washing brine WS1.2 | 53 ml | 310 | 27 | - |
| Washing brine WS2 | 59 ml | 310 | 7 | - |
| Brine Br1 for analysis | 100 ml | 296 | not detectable | 8.8 |
| Brine Br2 for analysis | 100 ml | 296 | not detectable | 8.8 |

Here too, it was found in the experiment that inorganic ions mostly remain in the mother liquor or can be removed by salt washing. The measured values are summarized in Table 8. Here, the masses of the ions in the 3 litres of the starting solution used were determined from the ion concentrations measured in the starting solution as for Table 6 above and entered in the table. The masses of the ions which would be present in the solid salt after corresponding double washing were calculated from the measured ion concentrations in brine Br2. For this purpose, the volume of the brine Br2 was converted according to the amount of solid salt: 100 ml / 30 g * 473 g = 1.577 ml.

**Table 8**

| **Ion** | **Sr** | **Ni** | **Ru** |
|---|---|---|---|
| **Unit** | **mg** | **mg** | **mg** |
| 3 l of starting solution (169 g/l of NaCl) | 0.17 | < 0.027 | < 0.073 |
| Brine Br2 (296 g/l of NaCl), converted to all the salt: 100 ml/ 30 g * 473 g = 1.577 ml | 0.124 | < 0.006 | < 0.017 |

### Example 8 (stage VI according to the invention)

### Crystallization after the prepurification of MDA process water, washing with deionized water:

MDA process water 2c (starting solution) after prepurification by means of the ozonolysis IIc is used and treated in a manner analogous to the procedure in **Example 2** (crystallization without prepurification of salt water from DPC production): about 94% of the water is withdrawn as distillate from the initial charge (MDA process water) with continual stirring. The remaining concentrate is separated on a suction filter into the mother liquor and solid salt (wet). A two-stage countercurrent wash using deionized water in the last washing stage is then carried out.

In the countercurrent wash, deionized water is brought into contact with the salt which has already been washed once, so that this salt is washed a second time. Here, part of the salt dissolves in the deionized water, which leads to a loss of solid salt. The salt-containing filtrate then formed from the deionized water is reused for the first washing of the salt. This in-principle procedure was approximated as follows:
The solid salt which had been separated off was divided into three approximately equal partial amounts (salt S1, salt S2 and salt S3). Salt S3 was washed with deionized water RW1 on the suction filter. The filtrate was collected as loaded washing water WW2. The loaded washing water WW2 thus represents an approximate of the filtrate which is reused for the first washing of the salt. However, this approximate is not very good since salt S3 had not yet been prewashed.

For this reason, salt S2 was then washed with the loaded washing water WW2 on the suction filter, resulting in loaded washing water WW3 as filtrate. The salt S2 which had been prewashed in this way was then washed with deionized water RW4 on the suction filter, giving loaded washing water WW5 as filtrate. This loaded washing water WW5 is then a significantly better approximate of a filtrate which is used for the first washing of the salt since it has been produced using prewashed salt S2.

Finally, salt S1 was washed with loaded washing water WW5 on the suction filter, giving loaded washing water WW6 as filtrate. The prewashed salt S1 was then washed with deionized water RW7 on the suction filter, giving loaded washing water WW8 as filtrate.

The washed salts S1, S2 and S3 were dried at about 100°C in a drying oven. For analytical purposes, 30 g of each of the dried salts S1, S2 and S3 were subsequently in each case taken up in deionized water to give 100 ml of solution, so that the brines Br1, Br2 and Br3 containing 300 g of NaCl/l were formed. The measured values for the various fractions are summarized in Table 9. The quality of the brines Br1, Br2 and Br3 produced in this way was comparatively very good. Owing to the prepurification according to the invention, only a small amount of TOC was found in the distillate (about 15% of the TOC burden) in the experiment.

**Table 9**

| **MDA after prepurification** | Amount | NaCl [g/l] | TOC [mg/l] | pH |
|---|---|---|---|---|
| Starting solution | 31 | 145 | 9.3 | 7.3 |
| Distillate | 2.6741 | 1.5 | 1.6 | 6.1 |
| Mother liquor | 155 ml | 314 | 91 | 9.6 |
| Solid salt (wet) | 406.5 g | - | - | - |
| Salt S1 (wet) | 135.8 g | - | - | - |
| Salt S2 (wet) | 135.2 g | - | - | - |
| Salt S3 (wet) | 135.5 g | - | - | - |
| Deionized water RW1 | 45 ml | - | - | - |
| Washing water WW2 | 50 ml | 258 | - | 9.3 |
| Washing water WW3 | 51 ml | 313 | 35.1 | 9.0 |
| Deionized water RW4 | 45 ml | - | - | - |
| Washing water WW5 | 49 ml | 304 | - | 9.1 |
| Washing water WW6 | 51 ml | 316 | 25 | 8.9 |
| Deionized water RW7 | 45 ml | - | - | - |
| Washing water WW8 | 51 ml | 304 | 13 | 8.8 |
| Brine Br1: 30 g of salt 1 + deionized water to 100 ml | 100 ml | 299 | < 1 | 8.0 |
| Brine Br2: 30 g of salt 2 + deionized water to 100 ml | 100 ml | 298 | < 1 | 8.1 |
| Brine Br3: 30 g of salt 3 + deionized water to 100 ml | 100 ml | 299 | 2.2 | 8.5 |

### Example 9 (stage VI according to the invention)

### Crystallization of a mixture of process water from MDA and DPC production:

A mixture of 80% of DPC process water 2b after prepurification by means of activated carbon and 20% MDA process water 2c after ozonolysis was used. 3 litres of the mixture were treated in a manner analogous to the procedure in **Example 2 (crystallization without purification DPC).** The measured values for the process materials are summarized in Table 9. As regards the removal of organic and inorganic impurities by crystallization and salt washing, the mixture behaves in a manner analogous to the behaviours of the individual process waters. 90% of the organic impurities are removed. Most impurities remain in the mother liquor; the remaining impurities are removed by the salt wash.

**Table 9**

| **Mixture:** | Amount | NaCl [g/l] | TOC [mg/l] | pH |
|---|---|---|---|---|
| **DPC 80%** | | | | |
| **MDA 20%** | | | | |
| Starting solution | 31 | 177 | 4 | 7.9 |
| Distillate | 2.6641 | 1.5 | 1.5 | 6.9 |
| Mother liquor | 129 ml | 310 | 37 | 9.4 |
| Solid salt (wet) | 516 g | - | - | - |
| Pure washing brine RW1 | 60 ml | 310 | < 1 | - |
| Pure washing brine RW2 | 60 ml | 310 | < 1 | - |
| Washing brine WS1.1 | 62 ml | 310 | - | - |
| Washing brine WS1.2 | 60 ml | 310 | 14 | - |
| Washing brine WS2 | 63 ml | 310 | 6 | - |
| Brine Br1 | 100 ml | 296 | < 1 | 9.3 |
| Brine Br2 | 100 ml | 296 | < 1 | 9.3 |

## Claims

1. Process for the work-up and reuse of salt-containing process water (la, lb, lc) from a production process (Ia, Ib, Ic), in particular from a chemical production process, which contains an alkali metal chloride, preferably sodium chloride, as salt in a concentration of at least 4% by weight and organic or inorganic and organic impurities, wherein
a) the process water (la, lb, lc) is firstly subjected to oxidative and/or adsorptive purification (IIa, IIb, IIc) to remove organic impurities, preferably with one or more adsorbents of the group consisting of: activated carbon, adsorbent resins and zeolites,
b) a preconcentrated, purified process water (4) is optionally produced from the purified process water (2a, 2b, 2c, 2d) by removal of water (3), to a concentration of not more than 26% by weight of alkali metal chloride in the process water, preferably as desired by means of one or more of the processes: high-pressure reverse osmosis, electrodialysis, evaporation, membrane distillation or vaporization,
c) a partial amount (6) of the purified process water (2a, 2b, 2c, 2d; 4) from step a) or b) having a salt concentration of from 4% by weight to 26% by weight, preferably from 7% by weight to 26% by weight, is introduced into the brine circuit (29) of a chloralkali electrolysis (VIII),
d) the residual amount of process water remaining from step c) is concentrated further by removal (V) of water
e) and the alkali metal chloride is crystallized (VI) out and
f) separated off as solid alkali metal chloride from the mother liquor and purified (VII) by means of a wash, so that the solid alkali metal chloride (13), analysed after dissolution in deionized pure water in a concentration of 300 g/l, has a TOC content of not more than 1 mg/l,
g) the solid, purified alkali metal chloride (13) from step f) is introduced into the brine stream (28) of the chloralkali electrolysis (VIII),
h) the products obtained from the alkali metal chloride electrolysis (VIII) after step g) and optionally c): chlorine (30), alkali metal hydroxide (31), preferably sodium hydroxide, and optionally hydrogen are optionally recirculated as desired to the production process (Ia, Ib, Ic).

2. Process according to Claim 1, **characterized in that** oxidative purification (IIa, IIb, IIc) in step a) for the removal of organic impurities is carried out by treatment with ozone at an initial pH to be set in the process water (la, lb, lc) of at least 1 and a temperature of at least 35°C, preferably at least 50°C, and the amount of ozone is preferably not more than 2 g of ozone/1 of process water (la, lb, lc).

3. Process according to either Claim 1 or 2, **characterized in that** the oxidative purification (IIc) of organic impurities from the process water (la, lb, lc) in step a) is carried out either in addition to another oxidative purification or solely by means of electrochemical anodic reaction at a diamond electrode, preferably at a boron-doped diamond electrode.

4. Process according to any of Claims 1 to 3, **characterized in that** the purification of organic impurities in step a) is carried out down to a residual content of impurities of not more than 5 mg/l of TOC.

5. Process according to any of Claims 1 to 4, **characterized in that** the concentration of salt, in particular of alkali metal chloride, in the process water (la, lb, lc) before step a) is at least 6% by weight, preferably at least 8% by weight, particularly preferably at least 12% by weight.

6. Process according to any of Claims 1 to 5, **characterized in that** the water (3) obtained in the preconcentration in the optional step b) is reused for diluting alkali metal hydroxide solution (33; 34), preferably sodium hydroxide solution, for the production process (Ia, Ib, Ic), in particular for a process for the preparation of polycarbonates (Ia), polycarbonate precursor (Ib) or MDA (Ic).

7. Process according to any of Claims 1 to 6, **characterized in that** the water (17, 18, 19, 23) obtained in the concentration and crystallization in the steps d) and e) is reused for diluting alkali metal hydroxide solution (33), preferably sodium hydroxide solution, for the production process (Ia, Ib, Ic), in particular a process for the preparation of polycarbonates, polycarbonate precursor or MDA.

8. Process according to any of Claims 1 to 7, **characterized in that** the solid alkali metal chloride (13) obtained in the crystallization (VI) in step f) is washed, preferably in countercurrent, by means of deionized water (16) and/or by means of purified alkali metal chloride solution (7) to effect purification before reuse.

9. Process according to any of Claims 1 to 8, **characterized in that** the production process (Ia, Ib, Ic) from which the process water (1a, lb, 1c) is taken is a process for the preparation of polycarbonates or of diphenyl carbonate, or of isocyanates, in particular methylenedi(phenylisocyanates), or of methylenedi(phenylamine) (MDA).

10. Process according to any of Claims 1 to 9, **characterized in that** water which is removed and is obtained in the preconcentration according to step b) and/or the concentration according to step d) or e) is used for washing of the solid salt (13) in step f).

11. Process according to any of Claims 1 to 10, **characterized in that** purified alkali metal chloride solution from a substream (7) of the process water which has been purified in step a) or from a substream of the purified process water which has been concentrated in step b) is used for the wash (VII) in step f).

12. Process according to any of Claims 1 to 11, **characterized in that** an alkali metal chloride solution for which purified alkali metal chloride salt is dissolved in water which is removed and obtained in the performance of step b) and/or d) is used for the wash (VII) in step f).

13. Process according to any of Claims 1 to 12, **characterized in that** the mother liquor which has been separated off from the alkali metal chloride (13) in step f) is divided into two streams, and the one larger stream is recirculated to the concentration according to step d) and the other smaller substream (12) which amounts to not more than 5% by weight of the mother liquor separated off is disposed of.

14. Process according to any of Claims 1 to 13, **characterized in that**, based on 100 parts by weight of alkali metal chloride separated off, from 5 to 20 parts by weight of washing liquid are used for washing of the solid alkali metal chloride in step f).

15. Process according to any of Claims 1 to 14, **characterized in that** the organic impurities are compounds selected from the group consisting of: aniline, MDA, formaldehyde, methanol, phenol; or from the group consisting of: bisphenol A, phenol and benzene compounds having different alkyl substitutions and also halogenated aromatics, preferably those from the group consisting of butylphenols, isopropylphenols, chlorophenols and bromophenols and also aliphatic amines and ammonium salts thereof, especially trimethylamines, butylamines, dimethylbenzylamines, ethylpiperidine and quaternary ammonium salts thereof.

16. Process according to any of Claims 1 to 15, **characterized in that** the inorganic impurities are compounds selected from the group consisting of: salts of cations of the metals: Ca, Mg, Fe, Al, Si, B, Sc, Ba, Ti, Cr, Mn, Ni and Ru in combination with anions, in particular anions selected from the group consisting of: Cl⁻ , F⁻, Br⁻, SiO₄²⁻, SO₄²⁻.

## Patentansprüche

1. Verfahren zur Aufbereitung und Wiederverwendung von salzhaltigem Prozesswasser (1a, 1b, 1c) aus einem Herstellungsverfahren (Ia, Ib, Ic), insbesondere aus einem chemischen Herstellungsverfahren, das ein Alkalichlorid, vorzugsweise Natriumchlorid, als Salz in einer Konzentration von mindestens 4 Gew.-% und organische oder anorganische und organische Verunreinigungen enthält, wobei
a) das Prozesswasser (1a, 1b, 1c) zuerst einer oxidativen und/oder adsorptiven Reinigung (IIa, IIb, IIc) zur Entfernung organischer Verunreinigungen unterzogen wird, vorzugsweise mit einem oder mehreren Adsorptionsmitteln aus der Gruppe bestehend aus: Aktivkohle, Adsorberharzen und Zeolithen,
b) gegebenenfalls aus dem gereinigten Prozesswasser (2a, 2b, 2c, 2d) durch Entfernen von Wasser (3) ein vorkonzentriertes, gereinigtes Prozesswasser (4) bis zu einer Konzentration von höchstens 26 Gew.-% Alkalichlorid im Prozesswasser hergestellt wird, vorzugsweise wahlweise durch eines oder mehrere der Verfahren: Hochdruck-Umkehrosmose, Elektrodialyse, Verdunstung, Membrandestillation oder Verdampfung,
c) gegebenenfalls eine Teilmenge (6) des gereinigten Prozesswassers (2a, 2b, 2c, 2d; 4) aus Schritt a) oder b) mit einer Salzkonzentration von 4 Gew.-% bis 26 Gew.-%, vorzugsweise von 7 Gew.-% bis 26 Gew.-%, dem Solekreislauf (29) einer Chloralkali-Elektrolyse (VIII) zugeführt wird,
d) die aus Schritt c) verbleibende Restmenge an Prozesswasser durch Entfernen (V) von Wasser weiter konzentriert wird,
e) und das Alkalichlorid auskristallisiert (VI) und
f) als festes Alkalichlorid von der Mutterlauge abgetrennt und mittels einer Wäsche gereinigt (VII) wird, sodass das feste Alkalichlorid (13), analysiert nach Lösung in vollentsalztem reinem Wasser mit einer Konzentration von 300 g/l, einen TOC-Gehalt von höchstens 1 mg/l aufweist,
g) das feste, gereinigte Alkalichlorid (13) aus Schritt f) dem Solestrom (28) der Chloralkali-Elektrolyse (VIII) zugeführt wird,
h) die aus der Alkalichlorid-Elektrolyse (VIII) nach Schritt g) und gegebenenfalls c) gewonnenen Produkte: Chlor (30), Alkalihydroxid (31), vorzugsweise Natriumhydroxid und gegebenenfalls Wasserstoff gegebenenfalls dem Herstellungsverfahren (Ia, Ib, Ic) wahlweise wieder zurückgeführt werden.

2. Verfahren gemäß Anspruch 1, **dadurch gekennzeichnet, dass** die oxidative Reinigung (IIa, IIb, IIc) in Schritt a) zur Entfernung organischer Verunreinigungen durch Behandlung mit Ozon bei einem einzustellenden AnfangspH-Wert des Prozesswassers (1a, 1b, 1c) von mindestens 1 und einer Temperatur von mindestens 35 °C, vorzugsweise mindestens 50 °C, erfolgt und wobei die Ozonmenge vorzugsweise höchstens 2 g Ozon/l Prozesswasser (1a, 1b, 1c) beträgt.

3. Verfahren gemäß Anspruch 1 oder 2, **dadurch gekennzeichnet, dass** die oxidative Reinigung (IIc) organischer Verunreinigungen aus dem Prozesswasser (1a, 1b, 1c) in Schritt a) entweder zusätzlich zu einer weiteren oxidativen Reinigung oder ausschließlich mittels elektrochemischer anodischer Umsetzung an einer Diamantelektrode, vorzugsweise an einer Bor-dotierten Diamantelektrode, erfolgt.

4. Verfahren gemäß einem der Ansprüche 1 bis 3, **dadurch gekennzeichnet, dass** die Reinigung organischer Verunreinigungen in Schritt a) bis zu einem Restgehalt an Verunreinigungen von höchstens 5 mg/1 TOC erfolgt.

5. Verfahren gemäß einem der Ansprüche 1 bis 4, **dadurch gekennzeichnet, dass** die Konzentration an Salz, insbesondere an Alkalichlorid im Prozesswasser (1a, 1b, 1c) vor Schritt a) mindestens 6 Gew.-%, vorzugsweise mindestens 8 Gew.-%, insbesondere vorzugsweise mindestens 12 Gew.-% beträgt.

6. Verfahren gemäß einem der Ansprüche 1 bis 5, **dadurch gekennzeichnet, dass** das in der Vorkonzentrierung im optionalen Schritt b) gewonnene Wasser (3) zur Verdünnung der Alkalilauge (33; 34), vorzugsweise Natronlauge, für das Herstellungsverfahren (Ia, Ib, Ic), insbesondere für ein Verfahren zur Herstellung von Polycarbonaten (Ia), Polycarbonatvorprodukt (Ib) oder MDA (Ic) weiterverwendet wird.

7. Verfahren gemäß einem der Ansprüche 1 bis 6, **dadurch gekennzeichnet, dass** das bei der Aufkonzentrierung und Kristallisation in den Schritten d) und e) gewonnene Wasser (17, 18, 19, 23) zur Verdünnung der Alkalilauge (33), vorzugsweise Natronlauge, für das Herstellungsverfahren (Ia, Ib, Ic), insbesondere ein Verfahren zur Herstellung von Polycarbonaten, Polycarbonatvorprodukt oder MDA, weiterverwendet wird.

8. Verfahren gemäß einem der Ansprüche 1 bis 7, **dadurch gekennzeichnet, dass** das bei der Kristallisation (VI) in Schritt f) gewonnene feste Alkalichlorid (13) vor einer Weiterverwendung mittels vollentsalztem Wasser (16) und/oder mittels gereinigter Alkalichloridlösung (7), vorzugsweise im Gegenstrom, zur Reinigung gewaschen wird.

9. Verfahren gemäß einem der Ansprüche 1 bis 8, **dadurch gekennzeichnet, dass** das Herstellungsverfahren (Ia, Ib, Ic), dem das Prozesswasser (1a, 1b, 1c) entnommen wird, ein Verfahren zur Herstellung von Polycarbonaten oder Diphenylcarbonat oder von Isocyanaten, insbesondere Methylendi(phenylisocyanaten), oder von Methylendi(phenylamin) (MDA) ist.

10. Verfahren gemäß einem der Ansprüche 1 bis 9, **dadurch gekennzeichnet, dass** zum Waschen des festen Salzes (13) in Schritt f) Wasser verwendet wird, das bei der Vorkonzentrierung gemäß Schritt b) und/oder der Aufkonzentrierung gemäß Schritt d) oder e) entfernt und gewonnen wird.

11. Verfahren gemäß einem der Ansprüche 1 bis 10, **dadurch gekennzeichnet, dass** für die Wäsche (VII) in Schritt f) gereinigte Alkalichloridlösung aus einem Teilstrom (7) des in Schritt a) gereinigten Prozesswassers oder aus einem Teilstrom des in Schritt b) aufkonzentrierten gereinigten Prozesswassers verwendet wird.

12. Verfahren gemäß einem der Ansprüche 1 bis 11, **dadurch gekennzeichnet, dass** für die Wäsche (VII) in Schritt f) eine Alkalichloridlösung verwendet wird, für die gereinigtes Alkalichlorid-Salz in Wasser gelöst wird, das bei der Durchführung von Schritt b) und/oder d) entfernt und gewonnen wird.

13. Verfahren gemäß einem der Ansprüche 1 bis 12, **dadurch gekennzeichnet, dass** die Mutterlauge, die in Schritt f) vom Alkalichlorid (13) abgetrennt wurde, in zwei Ströme geteilt wird, wobei der eine größere Strom in die Aufkonzentrierung gemäß Schritt d) zurückgeführt wird und der andere, höchstens 5 Gew.-% der abgetrennten Mutterlauge betragende kleinere Teilstrom (12), entsorgt wird.

14. Verfahren gemäß einem der Ansprüche 1 bis 13, **dadurch gekennzeichnet, dass** bezogen auf 100 Gewichtsteile abgetrenntes Alkalichlorid zum Waschen des festen Alkalichlorids in Schritt f) 5 bis 20 Gewichtsteile Waschflüssigkeit verwendet werden.

15. Verfahren gemäß einem der Ansprüche 1 bis 14, **dadurch gekennzeichnet, dass** die organischen Verunreinigungen Verbindungen sind, die aus der Gruppe bestehend aus: Anilin, MDA, Formaldehyd, Methanol, Phenol; oder aus der Gruppe bestehend aus: Bisphenol A, Phenol und Benzolverbindungen mit verschiedenen Alkylsubstitutionen und auch halogenierten Aromaten, vorzugsweise aus der Gruppe bestehend aus Butylphenolen, Isopropylphenolen, Chlorphenolen und Bromphenolen und auch aliphatischen Aminen und Ammoniumsalzen davon, insbesondere Trimethylaminen, Butylaminen, Dimethylbenzylaminen, Ethylpiperidin und quartären Ammoniumsalzen davon ausgewählt sind.

16. Verfahren gemäß einem der Ansprüche 1 bis 15, **dadurch gekennzeichnet, dass** die anorganischen Verunreinigungen Verbindungen sind, die aus der Gruppe bestehend aus: Salzen von Kationen der Metalle: Ca, Mg, Fe, Al, Si, B, Sc, Ba, Ti, Cr, Mn, Ni und Ru in Kombination mit Anionen, insbesondere Anionen, die aus der Gruppe bestehend aus: CL⁻, F⁻, Br⁻, SiO₄²⁻, SO₄²⁻ ausgewählt sind, ausgewählt sind.

## Revendications

1. Procédé de traitement et de valorisation d'une eau de procédé salée (1a, 1b, 1c) provenant d'un procédé de production (Ia, Ib, Ic), en particulier d'un procédé de production chimique, qui contient un chlorure de métal alcalin, de préférence du chlorure de sodium, comme sel en une concentration d'au moins 4 % en poids et des impuretés organiques ou inorganiques et organiques, dans lequel
a) l'eau de procédé (1a, 1b, 1c) est d'abord soumise à une purification par oxydation et/ou par adsorption (IIa, IIb, IIc) pour éliminer des impuretés organiques, de préférence avec un ou plusieurs adsorbants du groupe constitué par : carbone activé, résines adsorbantes et zéolithes,
b) une eau de procédé purifiée, préconcentrée (4) est éventuellement produite à partir de l'eau de procédé purifiée (2a, 2b, 2c, 2d) par élimination d'eau (3), jusqu'à une concentration ne dépassant pas 26 % en poids de chlorure de métal alcalin dans l'eau de procédé, de préférence au moyen d'un ou plusieurs des procédés : osmose inverse à haute pression, électrodialyse, évaporation, distillation membranaire ou vaporisation,
c) une quantité partielle (6) de l'eau de procédé purifiée (2a, 2b, 2c, 2d ; 4) de l'étape a) ou b) ayant une concentration en sel de 4 % en poids à 26 % en poids, de préférence de 7 % en poids à 26 % en poids, est introduite dans le circuit de saumure (29) d'une électrolyse chlore-alcali (VIII),
d) la quantité résiduelle d'eau de procédé restant de l'étape c) est concentrée davantage par élimination (V) d'eau
e) et le chlorure de métal alcalin est cristallisé (VI) et
f) séparé sous forme de chlorure de métal alcalin solide de la liqueur mère et purifié (VII) au moyen d'un lavage, de sorte que le chlorure de métal alcalin solide (13), analysé après dissolution dans de l'eau pure désionisée en une concentration de 300 g/l, a une teneur en COT ne dépassant pas 1 mg/l,
g) le chlorure de métal alcalin solide purifié (13) de l'étape f) est introduit dans le flux de saumure (28) de l'électrolyse chlore-alcali (VIII),
h) les produits issus de l'électrolyse de chlorure de métal alcalin (VIII) après l'étape g) et éventuellement c) : chlore (30), hydroxyde de métal alcalin (31), de préférence hydroxyde de sodium, et éventuellement hydrogène sont éventuellement recirculés comme souhaité dans le procédé de production (Ia, Ib, Ic).

2. Procédé selon la revendication 1, **caractérisé en ce que** la purification par oxydation (IIa, IIb, IIc) dans l'étape a) pour l'élimination d'impuretés organiques est effectuée par traitement avec de l'ozone à un pH initial à ajuster dans l'eau de procédé (1a, 1b, 1c) d'au moins 1 et à une température d'au moins 35 °C, de préférence d'au moins 50 °C, et la quantité d'ozone ne dépasse pas de préférence 2 g d'ozone/l d'eau de procédé (1a, 1b, 1c).

3. Procédé selon la revendication 1 ou 2, **caractérisé en ce que** la purification par oxydation (IIc) d'impuretés organiques de l'eau de procédé (1a, 1b, 1c) dans l'étape a) est effectuée soit en plus d'une autre purification par oxydation, soit uniquement par réaction anodique électrochimique au niveau d'une électrode en diamant, de préférence au niveau d'une électrode en diamant dopée au bore.

4. Procédé selon l'une quelconque des revendications 1 à 3, **caractérisé en ce que** la purification d'impuretés organiques dans l'étape a) est réduite jusqu'à obtention d'une teneur résiduelle en impuretés ne dépassant pas 5 mg/l de COT.

5. Procédé selon l'une quelconque des revendications 1 à 4, **caractérisé en ce que** la concentration en sel, en particulier en chlorure de métal alcalin, dans l'eau de procédé (1a, 1b, 1c) avant l'étape a) est d'au moins 6 % en poids, de préférence d'au moins 8 % en poids, de manière particulièrement préférée d'au moins 12 % en poids.

6. Procédé selon l'une quelconque des revendications 1 à 5, **caractérisé en ce que** l'eau (3) obtenue lors de la préconcentration dans l'étape facultative b) est réutilisée pour diluer une solution d'hydroxyde de métal alcalin (33 ; 34), de préférence une solution d'hydroxyde de sodium, pour le procédé de production (Ia, Ib, Ic), en particulier pour un procédé de préparation de polycarbonates (Ia), de précurseur de polycarbonate (Ib) ou de MDA (Ic).

7. Procédé selon l'une quelconque des revendications 1 à 6, **caractérisé en ce que** l'eau (17, 18, 19, 23) obtenue lors de la concentration et la cristallisation dans les étapes d) et e) est réutilisée pour diluer une solution d'hydroxyde de métal alcalin (33), de préférence une solution d'hydroxyde de sodium, pour le procédé de production (Ia, Ib, Ic), en particulier un procédé de préparation de polycarbonates, de précurseur de polycarbonate ou de MDA.

8. Procédé selon l'une quelconque des revendications 1 à 7, **caractérisé en ce que** le chlorure de métal alcalin solide (13) obtenu dans la cristallisation (VI) dans l'étape f) est lavé, de préférence à contre-courant, au moyen d'eau désionisée (16) et/ou au moyen d'une solution de chlorure de métal alcalin purifié (7) pour effectuer une purification avant valorisation.

9. Procédé selon l'une quelconque des revendications 1 à 8, **caractérisé en ce que** le procédé de production (Ia, Ib, Ic) duquel l'eau de procédé (1a, 1b, 1c) est prélevée est un procédé de préparation de polycarbonates ou de carbonate de diphényle, ou d'isocyanates, en particulier d'isocyanates de diphénylméthylène, ou de méthylènedi(phénylamine) (MDA).

10. Procédé selon l'une quelconque des revendications 1 à 9, **caractérisé en ce que** l'eau qui est éliminée et est obtenue dans la préconcentration selon l'étape b) et/ou la concentration selon l'étape d) ou e) est utilisée pour un lavage du sel solide (13) dans l'étape f).

11. Procédé selon l'une quelconque des revendications 1 à 10, **caractérisé en ce qu'**une solution de chlorure de métal alcalin purifié provenant d'un sous-flux (7) de l'eau de procédé qui a été purifiée dans l'étape a) ou d'un sous-flux de l'eau de procédé purifiée qui a été concentrée dans l'étape b) est utilisée pour le lavage (VII) dans l'étape f).

12. Procédé selon l'une quelconque des revendications 1 à 11, **caractérisé en ce qu'**une solution de chlorure de métal alcalin pour laquelle un sel de chlorure de métal alcalin purifié est dissous dans de l'eau qui est éliminée et obtenue dans la réalisation de l'étape b) et/ou d) est utilisée pour le lavage (VII) dans l'étape f).

13. Procédé selon l'une quelconque des revendications 1 à 12, **caractérisé en ce que** la liqueur mère qui a été séparée du chlorure de métal alcalin (13) dans l'étape f) est divisée en deux flux, le flux le plus grand est recirculé à la concentration selon l'étape d) et l'autre sous-flux plus petit (12), qui ne représente pas plus de 5 % en poids de la liqueur mère séparée, est éliminé.

14. Procédé selon l'une quelconque des revendications 1 à 13, **caractérisé en ce que**, sur la base de 100 parties en poids de chlorure de métal alcalin séparé, de 5 à 20 parties en poids de liquide de lavage sont utilisées pour un lavage du chlorure de métal alcalin solide dans l'étape f).

15. Procédé selon l'une quelconque des revendications 1 à 14, **caractérisé en ce que** les impuretés organiques sont des composés choisis dans le groupe constitué par : aniline, MDA, formaldéhyde, méthanol, phénol ; ou du groupe constitué par : bisphénol A, phénol et composés benzéniques ayant différentes substitutions par alkyle et également des composés aromatiques halogénés, de préférence ceux du groupe constitué par butylphénols, isopropylphénols, chlorophénols et bromophénols et également des amines aliphatiques et des sels d'ammonium correspondants, notamment les triméthylamines, butylamines, diméthylbenzylamines, l'éthylpipéridine et des sels d'ammonium correspondants.

16. Procédé selon l'une quelconque des revendications 1 à 15, **caractérisé en ce que** les impuretés inorganiques sont des composés choisis dans le groupe constitué par : sels de cations des métaux : Ca, Mg, Fe, Al, Si, B, Sc, Ba, Ti, Cr, Mn, Ni et Ru en combinaison avec des anions, en particulier des anions choisis dans le groupe constitué par : Cl⁻, F⁻, Br⁻, SiO₄²⁻, SO₄²⁻.
